# EUROPEAN PATENT APPLICATION

(11) **EP 4 736 762 A1**
(43) Date of publication of application: **06.05.2026**
(21) Application number: 24827710.5
(22) Date of filing: 11.06.2024
(51) Int. Cl.: A61B 5/151

(54) **BLOOD COLLECTION DEVICE**

(30) Priority: 28.06.2023 JP 2023106161
(71) Applicant: HITACHI HIGH-TECH CORPORATION, Tokyo 105-6409 (JP)
(72) Inventor: SUGIYAMA Kimikazu, Tokyo 105-6409 (JP); NUNOKAWA Hiroaki, Tokyo 105-6409 (JP)
(74) Representative: MERH-IP Matias Erny Reichl Hoffmann Patentanwälte PartG mbB
(86) International application number: PCT/JP2024/021211
(87) International publication number: WO 2025/004792

(57) **Abstract**

The present invention provides a blood collection device capable of efficiently and inexpensively performing puncture of a proper depth onto a finger of a person subjected to blood collection regardless of the properties of the finger. This blood collection device comprises: a puncture part (120) having a puncture needle (121); a puncture mechanism for moving the puncture part (120) and puncturing a finger (134) of a person subjected to blood collection with the puncture needle (121); and a control part for controlling the operation of the puncture mechanism. The puncture mechanism is a mechanism of lifting the puncture part (120) from below the finger (134) to puncture the finger (134) with the puncture needle (121). The control part controls the operation of the puncture mechanism to drive the rise and fall of the puncture part, and stops the rise of the puncture part (120) by the puncture mechanism by detecting the contact of the puncture part (120) with the finger (134).

## Description

### Technical Field

The present invention relates to a blood collection device for automatically collecting blood from a finger of a person subjected to blood collection.

### Background Art

In the related art, a blood collection device for automatically collecting blood from a finger of a person subjected to blood collection has been developed. As the blood collection device that automatically performs blood collection, there is a blood collection device in which a finger of a person subjected to blood collection is placed on a predetermined finger placement place, a puncture needle is automatically punctured into the finger, and blood flowing out from a puncture site is collected into a blood collection tube.

PTL 1 discloses a blood collection device that automatically collects blood from a finger of a person subjected to blood collection. This blood collection device is configured such that a perforator holder in which a puncture needle is built is lifted from below the finger so that a puncture needle punctures the finger. When the perforator holder is pressed by a push rod component, a perforator and a finger come into contact with each other, and the perforator receives a load equal to or greater than a certain level to puncture the finger.

PTL 2 discloses a blood test apparatus that punctures a finger with laser light and supplies blood to a blood sensor. The blood test apparatus includes a finger regulation member that regulates a position of the finger. By regulating the position of the finger with the finger regulation member, blood flowing out from the finger is easily stored in a reservoir.

PTL 3 discloses a body fluid collection aid used when collecting a body fluid from a puncture site on a living body surface. The body fluid collection aid is configured such that a puncture depth of a puncture needle to a fingertip is adjusted by a dial-type adjustment part. The puncture depth of the puncture needle to the fingertip is set to a puncture depth corresponding to an individual difference between persons subjected to blood collection and a puncture site.

### Citation List

### Patent Literature

PTL 1: JP2017-225519A
PTL 2: JP2009-089818A
PTL 3: JP2006-223320A

### Summary of Invention

### Technical Problem

In a blood collection device that automatically performs blood collection, when blood is collected from a finger of a person subjected to blood collection, it is necessary to puncture the finger with a puncture needle just enough. If the puncture is insufficient, it is necessary to ensure a sufficient puncture depth because the puncture does not reach a blood vessel or a required blood collection amount cannot be ensured. Meanwhile, if the puncture is excessive, a strong puncture pain may occur, so that a puncture depth more than necessary is to be avoided.

In the blood collection device that automatically performs blood collection, automatic movement of a puncture part to which a puncture needle is attached is controlled from an initial position to a position at which the finger of the person subjected to blood collection is punctured. In the blood collection device, a function of ensuring an appropriate puncture depth is required in order to perform puncture with just a right amount of depth. After the puncture part to which the puncture needle is attached is precisely moved from the initial position to a position close to a surface of the finger placed on a finger placement place, the puncture part needs to be pushed into the finger such that a tip of the puncture needle is within a predetermined puncture depth range.

As the blood collection device that automatically performs blood collection, there is a blood collection device that punctures a finger of a person subjected to blood collection with a puncture needle and automatically collects blood flowing down from a puncture site into a blood collection tube. In a case of such a method, a window-shaped opening penetrating up and down may be provided in the finger placement place. The finger of the person subjected to blood collection is placed on the finger placement place so that a ventral side is exposed downward from the opening. In the case of such a method, the puncture part to which the puncture needle is attached is lifted from below the finger placed on the finger placement place to perform puncture with the puncture needle. Therefore, the puncture part is driven to rise so that the tip of the puncture needle is within the predetermined puncture depth range.

However, there are individual differences in a size, a thickness, a distance from a surface to a blood vessel, a degree of swelling when blood is congested, and the like among fingers of persons subjected to blood collection. When the finger of the person subjected to blood collection is placed on a finger placement place provided with an opening, various individual differences also occur in a width in a vertical direction of fingers that protrude downward from the opening. In such a case, there is a problem that it is difficult to set the tip of the puncture needle attached to the puncture part within a predetermined puncture depth range.

In the technique disclosed in PTL 1, since the perforator holder is simply pushed up by the push rod component, there is room for improvement in regard to such a problem of individual differences. The technique disclosed in PTL 2 is for puncturing with a laser, and no countermeasure is taken against such a problem of individual differences. The technique disclosed in PTL 3 is for performing dial-type adjustment, and has an obstacle in application to a blood collection device that automatically performs blood collection.

As a method of adjusting the puncture depth, a method of sensing a distance to a blood vessel of a finger for each individual, a method of measuring a finger of a person subjected to blood collection in advance for each individual, or the like can be used. Based on these measurement results, a method of controlling the puncture part to which the puncture needle is attached to a target amount of movement according to the measurement result is conceivable. However, in such a method, there is a problem that labor of blood collection work and cost of equipment increase.

Therefore, an object of the invention is to provide a blood collection device capable of efficiently and inexpensively performing puncture of a proper depth onto a finger of a person subjected to blood collection regardless of properties of the finger.

### Solution to Problem

In order to solve the above problem, a blood collection device according to the invention is a blood collection device including: a puncture part having a puncture needle; a puncture mechanism configured to move the puncture part and puncture a finger of a person subjected to blood collection with the puncture needle; and a control part configured to control an operation of the puncture mechanism, in which the puncture mechanism is a mechanism that lifts the puncture part from below the finger to puncture the finger with the puncture needle, and the control part controls the operation of the puncture mechanism to drive rise and fall of the puncture part, and stops the rise of the puncture part by the puncture mechanism by detecting contact of the puncture part with the finger.

### Advantageous Effects of Invention

According to the invention, it is possible to provide the blood collection device capable of efficiently and inexpensively performing puncture of a proper depth onto the finger of the person subjected to blood collection regardless of properties of the finger.

### Brief Description of Drawings

[FIG. 1] FIG. 1 is an external view illustrating a blood collection device according to an embodiment of the invention.
[FIG. 2] FIG. 2 is a cross-sectional view illustrating a schematic example of a puncture mechanism of the blood collection device according to the embodiment of the invention.
[FIG. 3] FIG. 3 is a cross-sectional view illustrating an individual difference in a distance between a finger of a person subjected to blood collection and a puncture needle.
[FIG. 4A] FIG. 4A is a cross-sectional view illustrating an operation of a detection mechanism.
[FIG. 4B] FIG. 4B is a cross-sectional view illustrating an operation of the detection mechanism.
[FIG. 5A] FIG. 5A is a diagram illustrating an application example of the puncture mechanism and the detection mechanism.
[FIG. 5B] FIG. 5B is a diagram illustrating an application example of the puncture mechanism and the detection mechanism.
[FIG. 6A] FIG. 6A is a diagram illustrating an application example of the puncture mechanism and the detection mechanism.
[FIG. 6B] FIG. 6B is a diagram illustrating an application example of the puncture mechanism and the detection mechanism.

### Description of Embodiments

Hereinafter, a blood collection device according to an embodiment of the invention will be described. The same reference signs are given to the same components in the following drawings, and repeated description will be omitted.

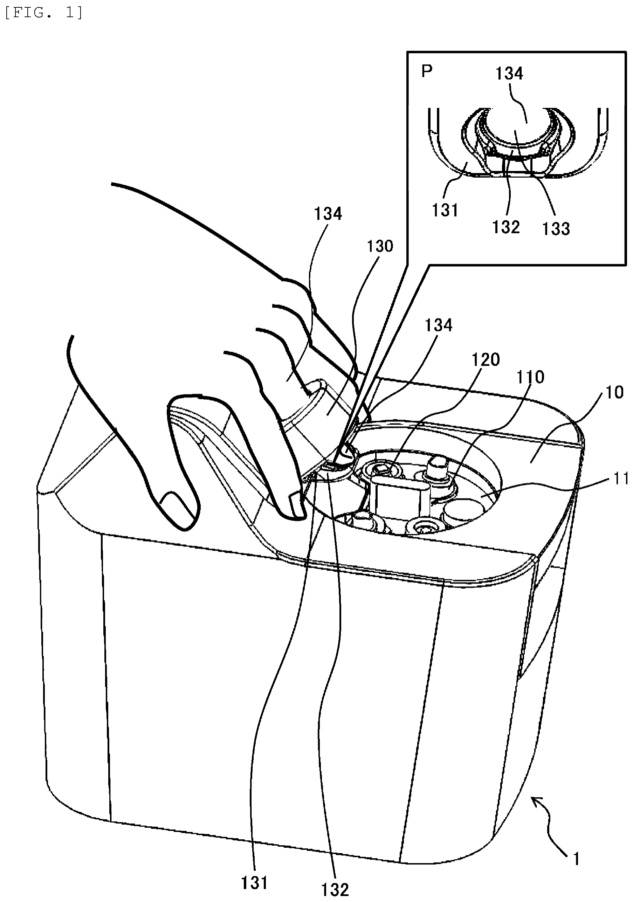

FIG. 1 is an external view illustrating the blood collection device according to the embodiment of the invention. FIG. 1 illustrates, as an example of the blood collection device, a finger blood collection device that automatically collects blood from a finger of a person subjected to blood collection. A reference sign P in FIG. 1 indicates an enlarged partial view of a periphery of a finger placement place of the blood collection device as viewed from below.

As illustrated in FIG. 1, a blood collection device 1 according to the present embodiment includes a housing 10, a turntable 11, a plurality of holders 110 that are installation locations for blood collection tubes, a plurality of modules 120 to which a puncture device, a hemostatic material, etc. can be attached, a cuff mechanism 130, a rotation drive mechanism (not illustrated) that rotates the turntable 11, a lifting drive mechanism (not illustrated) that lifts and lowers the holder 110 and the module 120, a pressure adjustment mechanism (not illustrated) that drives the cuff mechanism 130, and the like.

The housing 10 is formed of a plurality of structural materials, decorative plates, and the like. The turntable 11, the rotation drive mechanism, the lifting drive mechanism, the pressure adjustment mechanism, and the like are built in the housing 10. An upper surface of the housing 10 is provided with a hand placement place where a hand of a person subjected to blood collection is placed and a circular opening adjacent to the hand placement place. The turntable 11 is disposed below the opening.

The cuff mechanism 130 and a finger placement place 131 are provided on an opening side of the hand placement place. The cuff mechanism 130 is installed above the finger placement place 131 so as to surround a finger 134 of the person subjected to blood collection placed on the finger placement place 131. As illustrated in the partial view P, a disposable finger placement component 132 is attached to the finger placement place 131. A blood collection window 133 which is a through hole is opened in a window shape on a center side of the finger placement component 132. The finger 134 of the person subjected to blood collection is placed in the blood collection window 133 of the finger placement component 132.

The cuff mechanism 130 is a mechanism of fastening the finger 134 of the person subjected to blood collection (finger of the person subjected to blood collection) from surroundings. A cuff is placed on the finger placement place 131 so as to surround the placed finger 134 of the person subjected to blood collection. For example, the cuff is provided in a flexible bag shape, and is connected to a valve or a pump via a tube. The valve and the pump constitute the pressure adjustment mechanism that drives the cuff mechanism 130. Tightening pressure on the finger 134 of the person subjected to blood collection is adjusted by controlling internal pressure of the cuff by the pressure adjustment mechanism.

The turntable 11 is generally formed in a disk shape, and is supported inside the housing 10 such that a main surface faces upward and downward. The turntable 11 is provided with a plurality of portions for holding the holder 110 and the various modules 120. For example, a plurality of holding holes are provided penetrating the turntable 11 up and down. The holder 110 and the module 120 are held in the holding holes by being inserted vertically through the holding holes. Portions holding the holder 110 and the module 120 are regularly disposed at intervals along a circumferential direction of the turntable 11.

The holder 110 is an installation location for a blood collection tube, and various blood collection tubes such as a blood collection tube for a blood count test and a blood collection tube for biochemistry and immunological tests are installed in the holder 110. A blood collection tube having a predetermined size or an outer tube accommodating a blood collection tube can be installed in the holder 110. The outer tube is used for the purpose of adjusting a size of an installation object relative to an installation location of the blood collection tube.

The holder 110 and the module 120 can be detachably attached to the turntable 11. In the holder 110 and the module 120, for example, a flange-shaped portion having a diameter larger than an inner diameter of the holding hole of the turntable 11 is formed. The holder 110 and the module 120 can be held in a state where the holder 110 and the module 120 can move up and down in the holding hole by being inserted into the holding hole and supporting the flange-shaped portion from below.

For example, different types of modules such as a puncture module and a hemostatic module can be mounted as the module 120. A puncture device is attached to the puncture module. A puncture needle (lancet) is built in the puncture device. A hemostatic material such as gauze or a protective material such as an adhesive bandage is attached to the hemostatic module.

When the puncture device is pressed against a finger or the like of the person subjected to blood collection, the puncture needle protrudes to puncture the finger or the like of the person subjected to blood collection. A hemostatic material such as gauze is pressed against a puncture site of a person subjected to blood collection, and absorbs blood coming out from the puncture site to perform hemostasis. A protective material such as an adhesive bandage is pressed against a puncture site of a person subjected to blood collection so as to cover the puncture site, and performs sealing, hemostasis, and protection of the puncture site. The adhesive bandage is attached to the module 120 such that an adhesive surface faces upward.

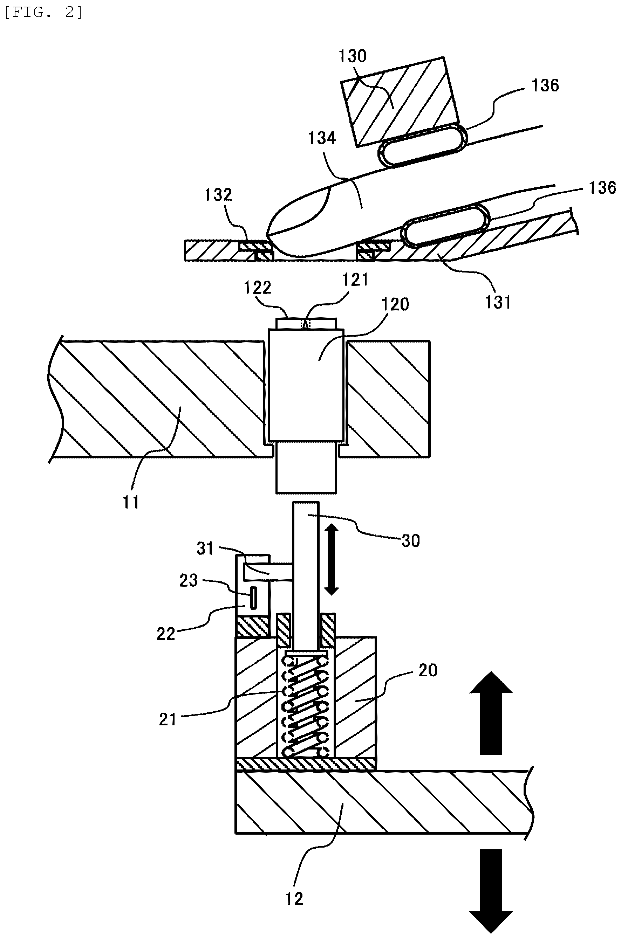

FIG. 2 is a cross-sectional view illustrating a schematic example of a puncture mechanism of the blood collection device according to the embodiment of the invention. FIG. 2 schematically illustrates a cross-sectional structure around the turntable 11 installed inside the blood collection device 1.

As illustrated in FIG. 2, in the blood collection device 1, the turntable 11, a lifting member 12, a base member 20, a movable support member 30, and the like are built in below the opening of the housing 10.

A compression coil spring 21 and a detection sensor 22 are attached to the base member 20. The movable support member 30 is supported on an upper end side of the compression coil spring 21. A detection target part 31 to be detected by the detection sensor 22 is formed on the movable support member 30. An upper end side of the movable support member 30 is a portion that pushes up the puncture module 120 and the like installed on the turntable 11.

A shaft (not illustrated) is connected to a center of the turntable 11. The other end of the shaft is coupled to an output shaft of a motor via a power transmission mechanism. The power transmission mechanism transmits rotational motion of the motor to the shaft by a predetermined mechanical mechanism. The turntable 11 is rotatable in both a clockwise direction and a counterclockwise direction with the shaft as a rotation axis by rotation of the shaft by the motor.

The turntable 11 is controlled to rotate by a predetermined step angle around the shaft as the rotation axis according to a blood collection operation or a treatment operation. The holder 110 and the module 120 are sequentially transported by the rotation of the turntable 11 to a blood collection position where the finger placement place 131 is formed.

At the blood collection position, puncturing with the puncture needle using the puncture module 120, hemostasis with gauze using the hemostatic module 120, and attachment of an adhesive bandage using the hemostatic module 120 are performed in this order. The finger 134 of the person subjected to blood collection is compressed by a cuff 136, and then punctured with the puncture needle by the puncture module 120. Blood flowing out from the puncture site is collected in the blood collection tube moved to the blood collection position on the turntable 11.

FIG. 2 illustrates a state in which the puncture module 120 is transported to the blood collection position. A puncture device 122 in which the puncture needle 121 is built is attached to the puncture module 120. The puncture module 120 constitutes a detachable puncture part having the puncture needle 121. By pushing up the puncture module 120, which is the puncture part, the puncture needle punctures the finger 134 of the person subjected to blood collection.

An operation of the puncture module 120 as the puncture part is driven by the puncture mechanism. The puncture mechanism is a mechanism that moves the puncture module 120, which is the puncture part, and punctures the finger 134 of the person subjected to blood collection with the puncture needle 121. The puncture mechanism lifts the puncture module 120, which is the puncture part, from below the finger 134 of the person subjected to blood collection to puncture the finger 134 of the person subjected to blood collection with the puncture needle 121. The puncture mechanism includes the lifting member 12, the base member 20, the movable support member 30, and the lifting drive mechanism that moves these members up and down.

The lifting member 12 is disposed below the blood collection position. The lifting member 12 is supported by the lifting drive mechanism (not illustrated) so as to be movable up and down. The lifting drive mechanism is implemented with a motor and a power transmission mechanism. The power transmission mechanism couples an output shaft of the motor and the lifting member 12 via a predetermined mechanical mechanism. Rotational motion of the motor is converted into up-down linear motion by the power transmission mechanism. The lifting member 12 is driven to move up and down by such a mechanism.

The base member 20 is supported by the lifting member 12. The base member 20 couples the lifting member 12 and the movable support member 30 and supports the compression coil spring 21 and the detection sensor 22. The base member 20 supports the compression coil spring 21 and the detection sensor 22 at a predetermined relative position with respect to the lifting member 12. The base member 20, the compression coil spring 21, the detection sensor 22, and the movable support member 30 can move up and down integrally with the lifting member 12.

The movable support member 30 rises integrally with the lifting member 12 to push up the holder 110 and the module 120 transported to the blood collection position from below. As the movable support member 30 rises, the puncture module 120, which is the puncture part, comes into contact with the finger 134 of the person subjected to blood collection, and the puncture needle punctures the finger 134 of the person subjected to blood collection. The blood collection tube held by the holder 110 or the module 120 to which the hemostatic material or the protective material is attached is pressed against the finger 134 of the person subjected to blood collection to perform treatment such as blood collection or hemostasis.

The blood collection device 1 includes a control part (not illustrated) that controls the operation of the puncture mechanism. The control part controls the operation of the puncture mechanism implemented with the lifting drive mechanism and the like to drive rise and fall of the puncture module 120, which is the puncture part, with respect to the finger placement place 131. The control part is implemented with a controller such as a programmable logic controller (PLC). The rise of the puncture module 120, which is the puncture part for the finger 134 of the person subjected to blood collection, is driven by a control input from the control part.

The compression coil spring 21 and the detection sensor 22, together with the detection target part 31 formed on the movable support member 30, form a detection mechanism that detects the puncture of the puncture needle 121 into the finger 134 of the person subjected to blood collection. The detection mechanism has a structure using elastic contraction by the compression coil spring 21, and is configured to detect puncture using a mechanical mechanism. In FIG. 2, the detection target part 31 is formed at an intermediate portion of the movable support member 30 in a vertical direction.

In the blood collection device 1 according to the present embodiment, the detection mechanism detects contact of the puncture module 120, which is the puncture part for the finger 134 of the person subjected to blood collection, and adjusts a puncture depth with respect to the finger 134 of the person subjected to blood collection within an appropriate range. When the puncture depth is adjusted within the appropriate range, even if there are individual differences in the finger 134 of the person subjected to blood collection, it is possible to perform puncturing with little excess or deficiency. Therefore, it is possible to stably ensure a required blood collection amount while avoiding puncture pain or the like due to puncture of the puncture needle 121.

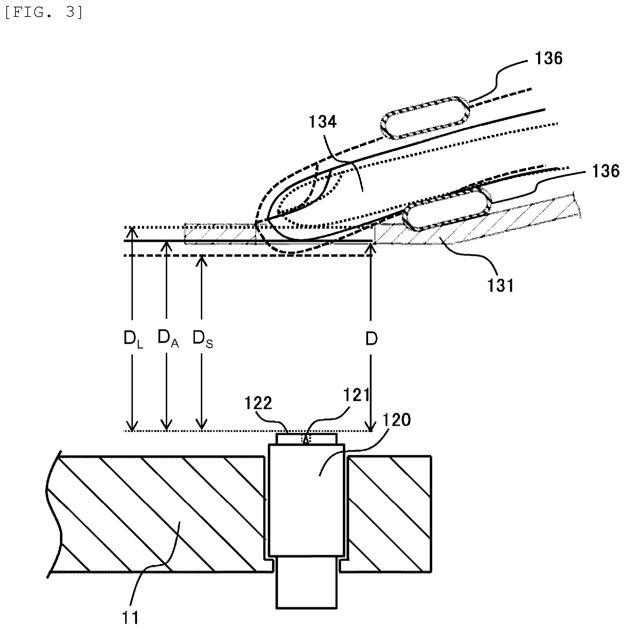

FIG. 3 is a cross-sectional view illustrating an individual difference in a distance between a finger of a person subjected to blood collection and a puncture needle. FIG. 3 schematically illustrates a cross-sectional structure around the turntable 11 installed inside the blood collection device 1, with some components omitted. In FIG. 3, solid lines indicating the finger 134 of the person subjected to blood collection indicate a position example of the finger when a size of the finger is standard. Broken lines indicate a position example when the size of the finger is larger than the standard. Dotted lines indicate a position example when the size of the finger is smaller than the standard.

Reference sign D indicates a distance between the finger placement place 131 and a reference position on the puncture module 120 stopped at an initial position. Examples of the reference position include a height position of an upper end of the module 120, which is a reference position for controlling an amount of movement.

Reference sign D_{A} denotes a distance between the finger when the size of the finger is standard and the reference position on the puncture module 120 at the initial position, reference sign D_{S} denotes a distance between the finger when the size of the finger is larger than the standard and the reference position on the puncture module 120 at the initial position, and reference sign D_{L} denotes a distance between the finger when the size of the finger is smaller than the standard and the reference position on the puncture module 120 at the initial position.

As illustrated in FIG. 3, the distance between the finger 134 of the person subjected to blood collection placed on the finger placement place 131 of the blood collection device 1 and the puncture needle 121 of the puncture module 120 at the initial position may differ for each person subjected to blood collection depending on the individual difference of the finger 134 of the person subjected to blood collection. Therefore, in order to adjust a puncture depth with respect to the finger 134 of the person subjected to blood collection within an appropriate range, it is necessary to appropriately control an amount of upward movement with respect to the finger 134 of the person subjected to blood collection with respect to the puncture module 120 which is the puncture part having the puncture needle 121.

As the puncture device 122, there is a type in which the puncture needle 121 is protruded by a spring. The puncture needle 121 is fixed to a holding hub built in the puncture device 122. A drive spring is built in below the puncture needle 121 and the holding hub. A pullback spring is built in above the puncture needle 121. The holding hub is configured to start deformation when the puncture device 122 comes into contact with the finger 134 of the person subjected to blood collection, and to release the puncture needle 121 when a predetermined deformation occurs.

In such a method, when the puncture device 122 comes into contact with the finger 134 of the person subjected to blood collection, the puncture needle 121 and the holding hub are pushed downward, and the drive spring elastically contracts. When the puncture needle 121 and the holding hub are further pushed down, the holding hub is deformed to release the puncture needle 121, and the puncture needle is pushed out by the drive spring to puncture the finger 134 of the person subjected to blood collection. The pushed-out puncture needle contracts the pullback spring and is pulled back downward by a restoring force of the pullback spring.

In a case of such a method, since an amount of protrusion of the puncture needle 121 is substantially constant, the puncture depth needs to be adjusted mainly by the amount of upward movement of the puncture module 120 which is the puncture part. When the puncture module 120 is lifted, an external force for operating at least the holding hub and the drive spring is required, and a minimum external force due to contact between the puncture device 122 attached to the puncture module 120 and the finger 134 of the person subjected to blood collection is required.

The finger 134 of the person subjected to blood collection has individual differences in size, thickness, and the like. A way in which the finger 134 of the person subjected to blood collection is placed on the finger placement place 131 of the blood collection device 1 and a response to compression by the cuff 136 may be different for each individual. When the finger 134 of the person subjected to blood collection is placed on the finger placement place 131 or compressed by the cuff 136, there are individual differences in a distance from a surface of a finger pad to a blood vessel, a degree of swelling when blood is congested, and the like.

As indicated by the broken line in FIG. 3, when the finger 134 of the person subjected to blood collection is thick, the distance D_{S} between the finger 134 of the person subjected to blood collection and the reference position on the puncture module 120 at the initial position is shorter than the standard distance D_{A}. In such a case, if the puncture depth is not appropriately adjusted, the puncture of the finger 134 of the person subjected to blood collection is likely to be insufficient. The puncture needle 121 may not reach the blood vessel, or damage to a blood vessel wall by the puncture needle 121 may be insufficient, and a required blood collection amount may not be ensured.

As indicated by the dotted line in FIG. 3, when the finger 134 of the person subjected to blood collection is thin, the distance D_{L} between the finger 134 of the person subjected to blood collection and the reference position on the puncture module 120 at the initial position is longer than the standard distance D_{A}. In such a case, unless the puncture depth is appropriately adjusted, the puncture to the finger 134 of the person subjected to blood collection is likely to be excessive. There is a concern that strong puncture pain may occur due to the puncture of the puncture needle 121, or it may be difficult to cure the puncture site.

In contrast, in the blood collection device 1 according to the present embodiment, the amount of movement by which the puncture module 120 is lifted from the initial position toward the finger 134 of the person subjected to blood collection is controlled to a predetermined amount of movement set in advance for a plurality of persons subjected to blood collection having individual differences in finger size, thickness, and the like. By controlling the amount of movement to be common for individuals, it is possible to ensure that the puncture is not insufficient, and a mechanical mechanism is used to prevent the puncture becoming excessive, and the puncture depth is efficiently adjusted within an appropriate range.

In the blood collection device 1 according to the present embodiment, the amount of movement by which the puncture module 120 is lifted from the initial position toward the finger 134 of the person subjected to blood collection is set to an amount of movement (D + α) that is larger by a predetermined amount α than the distance D between the finger placement place 131 and the reference position on the puncture module 120 at the initial position. In the control part, the target amount of movement (D + α) to which a margin of the predetermined amount α is added is set in advance as a control target value of the puncture mechanism.

In the blood collection device 1 according to the present embodiment, the rise of the puncture module 120 with respect to the finger 134 of the person subjected to blood collection is stopped by detecting contact of the puncture module 120 with the finger 134 of the person subjected to blood collection. The control part controls the lifting drive mechanism of lifting the puncture module 120, which is the puncture part, so as to achieve the preset target amount of movement (D + α), but stops lifting the puncture module 120 by detecting contact of the puncture module 120 with the finger 134 of the person subjected to blood collection.

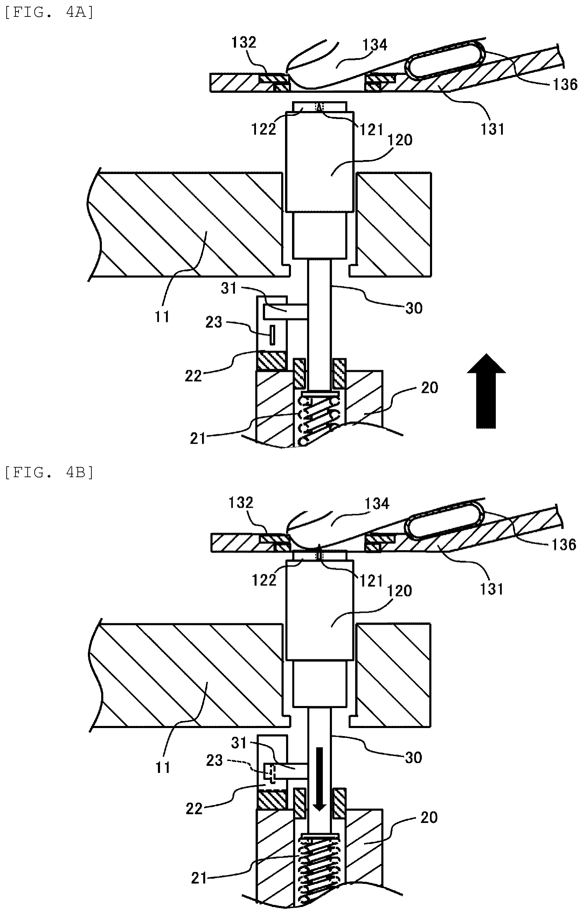

FIGS. 4A and 4B are cross-sectional views illustrating an operation of the detection mechanism. FIGS. 4A and 4B schematically illustrate the operation of the detection mechanism that detects contact of the puncture module 120 with the finger 134 of the person subjected to blood collection, with some components around the turntable 11 omitted. FIG. 4A illustrates a state before the puncture module 120 comes into contact with the finger 134 of the person subjected to blood collection. FIG. 4B illustrates a state after the puncture module 120 comes into contact with the finger 134 of the person subjected to blood collection.

As illustrated in FIGS. 4A and 4B, the contact of the puncture module 120 with the finger 134 of the person subjected to blood collection is detected by the compression coil spring 21 which is an elastic member, the detection sensor 22, and the detection target part 31 constituting the detection mechanism. The detection target part 31 can be formed in an appropriate shape such as a plate shape or a rod shape as a non-light-transmitting member.

The compression coil spring 21 elastically supports the movable support member 30 so that the movable support member 30 can move up and down relative to the base member 20. The compression coil spring 21 is disposed such that a direction of expansion and contraction is parallel to the vertical direction. A relative position of a lower end side of the compression coil spring 21 is fixed with respect to a lifting member 21 side. An upper end side of the compression coil spring 21 is fixed to a lower end side of the movable support member 30. The compression coil spring 21 biases the movable support member 30 upward, and elastically contracts in the vertical direction when the movable support member 30 receives a downward force.

The compression coil spring 21 is provided with a spring constant that is balanced with a load due to weight of the movable support member 30 in elastic displacement in an initial state, but contracts when the puncture module 120 receives an external force from the finger 134 of the person subjected to blood collection. When the puncture device 122 is of a type in which the drive spring protrudes, the compression coil spring 21 needs to have a spring constant such that the compression coil spring 21 contracts with an external force larger than an external force with which the holding hub or the drive spring operates.

The detection sensor 22 detects that the detection target part 31 moves to a predetermined position. A relative position of the detection sensor 22 is fixed with respect to the base member 20 and the lifting member 12. The detection sensor 22 is disposed in the vicinity of the movable support member 30 and below the detection target part 31 that moves up and down. The detection sensor 22 can be supported on an upper portion or the like of the base member 20.

In FIGS. 4A, 4B, and the like, a photo interrupter is provided as the detection sensor 22. The photo interrupter includes a light emitting element and a light receiving element as a detection part 23 that detects the detection target part 31. The light emitting element and the light receiving element are disposed on a lower side of upper and lower tracks of the detection target part 31 so as to face each other at a predetermined height. A gap is formed between the light emitting element and the light receiving element so that the detection target part 31 formed on the movable support member 30 can move forward and backward.

The light emitting element of the photo interrupter is formed of a light emitting diode or the like that emits infrared light or the like. The light receiving element is formed of a photodiode, a phototransistor, or the like that detects light emission of the light emitting element. When the detection target part 31 enters between the light emitting element and the light receiving element, light from the light emitting element to the light receiving element is blocked, and a photocurrent due to received light decreases, so that a non-light-transmitting detection target is detected.

According to the detection sensor 22, when the movable support member 30 receives a downward force and the compression coil spring 21 is compressed, it is possible to detect that the detection target part 31 is lowered to a predetermined height. Therefore, it is possible to indirectly detect that the puncture module 120 comes into contact with the finger 134 of the person subjected to blood collection by the detection of the detection target part 31. Since the amount of movement of the puncture module 120 can be limited without sensing the distance by an optical sensor or the like, cost of the device can be reduced as compared with a method of sensing the distance to the blood vessel of the finger.

As illustrated in FIG. 4A, when the puncture needle 121 punctures the finger 134 of the person subjected to blood collection, the base member 20 and the lifting member 12 are driven to rise. As the base member 20 is lifted, the movable support member 30 supported by the base member 20 is also lifted, and the puncture module 120, which is the puncture part, is pushed up to a height at which the module 120 comes into contact with the finger 134 of the person subjected to blood collection placed on the finger placement place 131.

As illustrated in FIG. 4A, before the puncture module 120 comes into contact with the finger 134 of the person subjected to blood collection, the compression coil spring 21 is in an extended state, and a relative position of the movable support member 30 with respect to the base member 20 is on an upper side. The detection target part 31 formed on the movable support member 30 is located above the detection part 23 and is not detected by the detection sensor 22.

Meanwhile, as illustrated in FIG. 4B, when the puncture module 120 comes into contact with the finger 134 of the person subjected to blood collection, the puncture needle 121 protrudes from the puncture device 122 and punctures the finger 134 of the person subjected to blood collection. The puncture of the puncture needle 121 with respect to the finger 134 of the person subjected to blood collection is performed in a state where the movable support member 30 is biased in an upward direction by the compression coil spring 21. Therefore, when the puncture device 122 is of a type in which the drive spring protrudes, an external force for operating the holding hub and the drive spring can be easily ensured.

As illustrated in FIG. 4B, when the puncture module 120 comes into contact with the finger 134 of the person subjected to blood collection, the module 120 receives a downward external force from the finger 134 of the person subjected to blood collection. By such an external force, the puncture module 120 and the movable support member 30 do not rise, and the compression coil spring 21 contracts. After the puncture part comes into contact with the finger 134 of the person subjected to blood collection, the compression coil spring 21 is compressed, and the relative position of the movable support member 30 with respect to the base member 20 moves downward. The detection target part 31 formed on the movable support member 30 is lowered to a height of the detection part 23 and detected by the detection sensor 22.

With such a detection mechanism, while the base member 20 and the lifting member 12 are driven to rise, contact of the puncture module 120, which is the puncture part, with the finger 134 of the person subjected to blood collection is detected. When the contact of the puncture module 120, which is the puncture part, with the finger 134 of the person subjected to blood collection is detected, a detection signal is transmitted from the detection sensor 22 to the control part, and the rise of the lifting member 12 by the control part is stopped. By stopping the rise of the lifting member 12, excessive puncture of the finger 134 of the person subjected to blood collection can be prevented.

The amount of movement by which the puncture module 120 is lifted from the initial position toward the finger 134 of the person subjected to blood collection can be controlled to the constant amount of movement (D + α) set in advance for a plurality of persons subjected to blood collection having individual differences in finger size, thickness, and the like. As the predetermined amount α, a length by which the amount of movement (D + α) is longer than the standard distance D_{A} is set so that insufficient puncture due to individual differences does not occur even when the finger 134 of the person subjected to blood collection is thin or the like. For example, a margin longer than a difference between the distance D_{L} corresponding to a predetermined standard deviation and the standard distance D_{A} can be set.

In general, as a method of adjusting a puncture depth in blood collection from a finger having an individual difference, there is a method of controlling an amount of movement of a puncture needle with respect to a finger of a person subjected to blood collection to a target amount of movement that matches an actually measured measurement result. Examples of the measurement for setting the target amount of movement include a method of measuring the distance between the puncture needle and the blood vessel of the finger of the person subjected to blood collection with a distance measuring sensor or the like for each individual, and a method of manually measuring the size of the finger of the person subjected to blood collection in advance for each individual.

However, when the distance between the puncture needle and the blood vessel of the finger of the person subjected to blood collection is measured by a distance measuring sensor or the like, an optical sensor or the like is required, and there is a problem that the cost of the device increases. When the measurement is manually performed in advance, it is necessary to perform manual measurement for each person subjected to blood collection, input work of a measurement result, and the like, and there is a problem that labor of blood collection work increases.

In contrast, in the blood collection device 1 according to the present embodiment, since the contact of the puncture module 120, which is the puncture part, with the finger 134 of the person subjected to blood collection is detected by the detection mechanism using the mechanical mechanism, the puncture depth with respect to the finger 134 of the person subjected to blood collection can be adjusted efficiently and inexpensively within a range with little excess or deficiency. Since the target amount of movement (D + α) to which the margin of the predetermined amount α is added is set in advance, and the movement of the puncture part is stopped at a time when the contact of the puncture module 120, which is the puncture part, with the finger 134 of the person subjected to blood collection is detected, even when there is an individual difference in the finger 134 of the person subjected to blood collection, it is possible to stably ensure a necessary blood collection amount while avoiding puncture pain or the like due to puncture of the puncture needle 121. Since appropriate blood collection can be automatically performed, it is possible to obtain the blood collection device 1 having high reliability that automatically collects blood from the finger of the person subjected to blood collection.

The contact of the puncture module 120, which is the puncture part, with the finger 134 of the person subjected to blood collection is detected by detecting the lowering of the movable support member 30 against bias of the compression coil spring 21, which is an operation caused by the contact of the puncture module 120, which is the puncture part, with the finger. Therefore, the contact of the puncture module 120 with the finger 134 of the person subjected to blood collection can be detected using the mechanical mechanism by adjusting the spring constant of the compression coil spring 21.

The lowering of the movable support member 30 is detected by the detection target part 31 that moves up and down together with the movable support member 30 with respect to the base member 20 and the detection sensor 22 that detects that the detection target part 31 moves to a predetermined position. Therefore, the operation using the mechanical mechanism by the compression coil spring 21 can be detected by a low-cost device as compared with an optical displacement sensor or the like.

In FIGS. 4A, 4B, and the like, a photo interrupter is provided as the detection sensor 22, but a mechanical switch such as a micro switch, a non-contact proximity sensor using an eddy current, magnetism, electromagnetic induction, or the like, may be used as the detection sensor 22. These detection sensors 22 can be used to detect the detection target part 31 that is lowered against the bias of the compression coil spring 21.

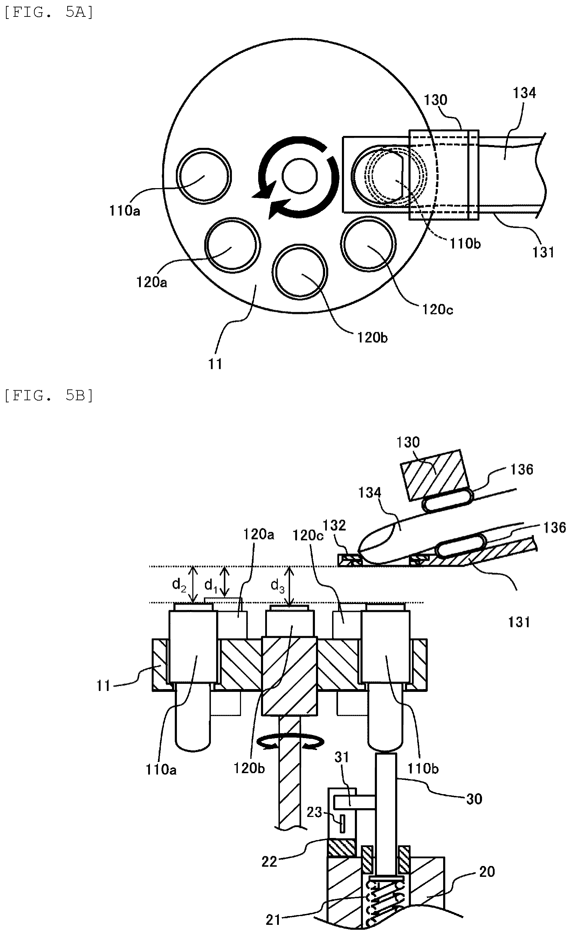

FIGS. 5A and 5B are diagrams illustrating application examples of the puncture mechanism and the detection mechanism. FIGS. 5A and 5B schematically illustrate the application examples in which the puncture mechanism and the detection mechanism are applied to the turntable 11, with some components around the turntable 11 omitted. FIG. 5A is a plan view of the periphery of the turntable 11 as viewed from above. FIG. 5B is a partial cross-sectional view of the periphery of the turntable 11 as viewed from a side.

As illustrated in FIGS. 5A and 5B, a plurality of holders 110 for holding blood collection tubes and a plurality of modules 120 can be attached to the blood collection device 1. The puncture mechanism and the detection mechanism can also be used to adjust the amount of movement of the finger 134 of the person subjected to blood collection in a blood collection operation using the holder 110 on the turntable 11 or a treatment operation using the module 120 other than the puncture module 120.

In FIGS. 5A and 5B, as the holder 110 for holding a blood collection tube, a first holder 110a for holding a first blood collection tube and a second holder 110b for holding a second blood collection tube are installed on the turntable 11. As these blood collection tubes, for example, a blood collection tube coated with an anticoagulant for a blood count test, or a blood collection tube containing a separating agent for biochemistry and immunological tests can be installed.

In FIGS. 5A and 5B, as the module 120, a puncture module 120a which is a puncture part, a hemostatic module 120b that holds a protective material such as an adhesive bandage, and a hemostatic module 120c that holds a hemostatic material such as gauze are installed on the turntable 11.

In the blood collection operation using the holder 110 or the treatment operation using the module 120 other than the puncture module 120, when the amount of movement with respect to the finger 134 of the person subjected to blood collection is adjusted, the puncture mechanism functions as a movement mechanism that moves the holder 110 or the module 120 to press the blood collection tube, the hemostatic material, or the protective material against the finger of the person subjected to blood collection. The movement mechanism includes the lifting member 12, the base member 20, the movable support member 30, and the lifting drive mechanism that moves these members up and down. The operation of the movement mechanism including the lifting drive mechanism and the like is controlled by the control part implemented with a controller.

As illustrated in FIG. 5A, the holder 110 and the module 120 are sequentially transported to the blood collection position where the finger placement place 131 is installed by rotation of the turntable 11, and then are driven to rise by being pushed up by the movable support member 30. As the holder 110 is lifted, the blood collection tube is pressed against the finger 134 of the person subjected to blood collection, and the blood flowing out from the puncture site is collected. As the module 120 is lifted, the protective material or the hemostatic material is pressed against the finger 134 of the person subjected to blood collection, and blood flowing out from the puncture site is stopped.

As illustrated in FIG. 5B, the holder 110 and the module 120 may have different heights. In FIG. 5B, reference sign d₁ denotes a distance between the finger placement place 131 and a reference position on the puncture module 120a at an initial position, reference sign d₂ denotes a distance between the finger placement place 131 and a reference position on the blood collection tube installed in the holder 110a at an initial position, and reference sign d₃ denotes a distance between the finger placement place 131 and a reference position on the hemostatic module 120b at an initial position. As an example, these distances have a relationship of d₁ < d₂ < d₃.

In the blood collection device 1, the amount of movement of the puncture module 120, which is the puncture part, can be controlled to be the preset target amount of movement (D + α). Meanwhile, the amount of movement of the holder 110 and the module 120 other than the puncture module 120, which is the puncture part, can be set to match the distance between the finger 134 of the person subjected to blood collection and the reference position on the holder 110 or the module 120 at the initial position based on a measurement result of the amount of movement of the puncture module 120 measured when the puncture module 120 is lifted.

In the blood collection device 1, first, a puncture operation is performed by lifting and lowering the puncture module 120a, which is the puncture part, among the plurality of holders 110 and the plurality of modules 120. After the puncture module 120 is driven to rise so as to achieve the preset target amount of movement (D + α), the rise is stopped at a time when the contact with the finger 134 of the person subjected to blood collection is detected.

While the puncture operation is performed, an actual amount of movement of the puncture module 120 until the puncture module 120 comes into contact with the finger 134 of the person subjected to blood collection and stops can be measured by a displacement sensor or the like that measures a displacement amount of the movable support member 30. Data of the actual amount of movement of the puncture module 120 is stored in a memory or the like of the blood collection device 1.

Subsequently, a blood collection operation is performed by lifting and lowering the holder 110 in which the blood collection tube is installed. The holder 110 in which the blood collection tube is installed is transported to the blood collection position where the finger placement place 131 is installed by the rotation of the turntable 11, and then is driven to rise by being pushed up by the movable support member 30.

The amount of movement of the holder 110 in which the blood collection tube is installed can be set to a target amount of movement corresponding to the height of the holder 110 based on the measurement result of the amount of movement of the puncture module 120 measured when the puncture module 120 is lifted. The control part can acquire the data of the actual amount of movement of the puncture module 120 measured during the puncture operation, correct the data based ondata of the height of the holder 110 specified in advance, and output a control target value of the amount of movement of the holder 110 to the movement mechanism.

For example, a difference (d₂ - d₁) between the distance d₂ between the finger placement place 131 and the reference position on the blood collection tube installed in the holder 110a at the initial position and the distance d₁ between the finger placement place 131 and the reference position on the puncture module 120a at the initial position can be set to a target amount of movement added to the actual amount of movement of the puncture module 120.

Subsequently, a treatment operation is performed by lifting and lowering the hemostatic module 120 to which the hemostatic material is attached. The treatment operation is performed by lifting and lowering the hemostatic module 120 to which the protective material is attached. The hemostatic module 120 is transported to the blood collection position where the finger placement place 131 is installed by the rotation of the turntable 11, and then is driven to rise by being pushed up by the movable support member 30.

The amount of movement of the hemostatic module 120 can be set to a target amount of movement corresponding to a height of the hemostatic module 120 based on the measurement result of the amount of movement of the puncture module 120 measured when the puncture module 120 is lifted. The control part can acquire the data of the actual amount of movement of the puncture module 120 measured during the puncture operation, correct the data based on data of the height of the hemostatic module 120 specified in advance, and output a control target value of the amount of movement of the hemostatic module 120 to the movement mechanism.

For example, a difference (d₃ - d₁) between the distance d₃ between the finger placement place 131 and the reference position on the hemostatic module 120b at the initial position and the distance d₁ between the finger placement place 131 and the reference position on the puncture module 120a at the initial position can be set to a target amount of movement added to the actual amount of movement of the puncture module 120.

According to such a blood collection device 1, since the amount of movement of the holder 110 and the module 120 other than the puncture module 120 which is the puncture part is set based on the measurement result of the amount of movement of the puncture module 120, it is possible to appropriately perform treatment such as blood collection or hemostasis after puncture even when there is an individual difference in the finger 134 of the person subjected to blood collection and the holder 110 and the module 120 have different heights. Since the blood collection tube, the hemostatic material, or the protective material can be appropriately pressed against the finger 134 of the person subjected to blood collection, excessive pressing, or insufficient pressing against the finger 134 of the person subjected to blood collection can be avoided.

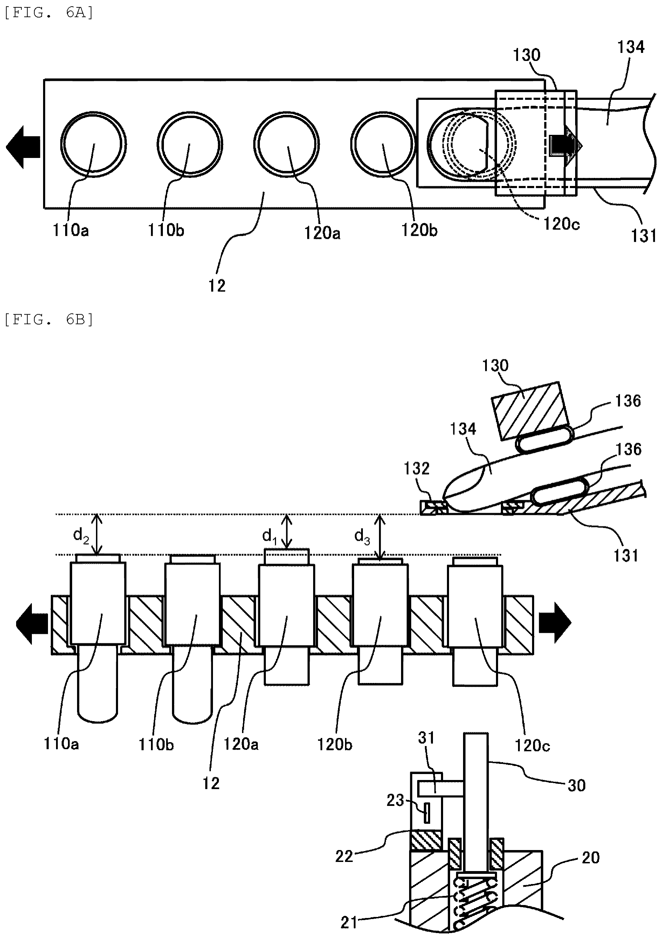

FIGS. 6A and 6B are diagrams illustrating application examples of the puncture mechanism and the detection mechanism. FIGS. 6A and 6B schematically illustrate the application examples in which the puncture mechanism and the detection mechanism are applied to a rack in which the holder 110 and the module 120 are installed, with some of peripheral components omitted. FIG. 6A is a plan view of a periphery of the rack 12 as viewed from above. FIG. 6B is a partial cross-sectional view of the periphery of the rack 12 as viewed from a side.

As illustrated in FIGS. 6A and 6B, the blood collection device 1 may include the rack 12 instead of the turntable 11 as a place where the holder 110 and the module 120 are installed. The puncture mechanism and the detection mechanism can also be used to adjust the amount of movement of the finger 134 of the person subjected to blood collection in a puncture operation performed on the rack 12, a blood collection operation using the holder 110, or a treatment operation using the module 120 other than the puncture module 120.

The rack 12 is provided in a line shape having a substantially rectangular parallelepiped shape. The rack 12 is provided with a plurality of parts that support the holders 110, which are installation locations for blood collection tubes, and various modules 120. The parts that support the holders 110 and the modules 120 are regularly arranged at intervals along a longitudinal direction of the rack 12.

The rack 12 is movable in both directions parallel to the longitudinal direction by a drive mechanism (not illustrated). A step operation of the rack 12 with respect to a blood collection position where the finger placement place 131 is formed is controlled according to the blood collection operation or the treatment operation. The holder 110 and the module 120 are sequentially transported by the movement of the rack 12 to the blood collection position where the finger placement place 131 is formed. The puncture mechanism and the detection mechanism can be installed below the rack 12 as in a case of the turntable 11.

In FIGS. 6A and 6B, as the holder 110 for holding a blood collection tube, the first holder 110a for holding the first blood collection tube and the second holder 110b for holding the second blood collection tube are installed on the rack 12. As these blood collection tubes, for example, a blood collection tube coated with an anticoagulant for a blood count test, or a blood collection tube containing a separating agent for biochemistry and immunological tests can be installed.

In FIGS. 6A and 6B, as the module 120, the puncture module 120a which is the puncture part, the hemostatic module 120b that holds a protective material such as an adhesive bandage, and the hemostatic module 120c that holds a hemostatic material such as gauze are installed on the rack 12.

In the blood collection operation using the holder 110 or the treatment operation using the module 120 other than the puncture module 120, when the amount of movement with respect to the finger 134 of the person subjected to blood collection is adjusted, the puncture mechanism functions as a movement mechanism that moves the holder 110 or the module 120 to press the blood collection tube, the hemostatic material, or the protective material against the finger of the person subjected to blood collection. The movement mechanism includes the lifting member 12, the base member 20, the movable support member 30, and the lifting drive mechanism that moves these members up and down.

As illustrated in FIG. 6A, the holder 110 and the module 120 are sequentially transported to the blood collection position where the finger placement place 131 is installed by movement of the rack 12, and then are driven to rise by being pushed up by the movable support member 30. As the holder 110 is lifted, the blood collection tube is pressed against the finger 134 of the person subjected to blood collection, and the blood flowing out from the puncture site is collected. As the module 120 is lifted, the protective material or the hemostatic material is pressed against the finger 134 of the person subjected to blood collection, and blood flowing out from the puncture site is stopped.

As illustrated in FIG. 6B, the holder 110 and the module 120 may have different heights. In FIG. 6B, the reference sign d₁ denotes the distance between the finger placement place 131 and the reference position on the puncture module 120a at the initial position, the reference sign d₂ denotes the distance between the finger placement place 131 and the reference position on the blood collection tube installed in the holder 110a at the initial position, and the reference sign d₃ denotes the distance between the finger placement place 131 and the reference position on the hemostatic module 120b at the initial position. As an example, these distances have a relationship of d₁ < d₂ < d₃.

When the rack 12 is provided, the amount of movement of the puncture module 120, which is the puncture part, can be controlled to be the preset target amount of movement (D + α) as in the case of the turntable 11. The amount of movement of the holder 110 and the module 120 other than the puncture module 120, which is the puncture part, can be set to match the distance between the finger 134 of the person subjected to blood collection and the reference position on the holder 110 or the module 120 at the initial position based on a measurement result of the amount of movement of the puncture module 120 measured when the puncture module 120 is lifted.

According to such a blood collection device 1, since the holder 110 and the module 120 can be transported in one axial direction by the rack 12, a blood collection operation at a blood collection position, a blood measurement operation at a position other than the blood collection position, and the like can be efficiently performed at each location. Since a peripheral device or wiring does not interfere with a shaft or the like or is not affected by the rotation of the turntable 11, a degree of freedom in installation or wiring of the device may be improved.

Although the embodiment of the invention has been described above, the invention is not limited to the embodiment described above, and various modifications can be made without departing from the gist of the invention. For example, the invention is not necessarily limited to one including all the configurations included in the above-described embodiment. A part of a configuration of an embodiment may be replaced with another configuration, a part of the configuration of the embodiment may be added to another configuration, or a part of the configuration of the embodiment may be omitted.

### Reference Signs List

- 10:: housing
- 11:: turntable
- 12:: rack
- 12:: lifting member
- 20:: base member
- 21:: compression coil spring
- 22:: detection sensor
- 23:: detection part
- 30:: movable support member
- 31:: detection target part
- 110:: holder
- 120:: module
- 121:: puncture needle
- 122:: puncture device
- 130:: cuff mechanism
- 131:: finger placement place
- 132:: finger placement component
- 133:: blood collection window
- 134:: finger of person subjected to blood collection
- 136:: cuff

## Claims

1. A blood collection device comprising:
a puncture part having a puncture needle;
a puncture mechanism configured to move the puncture part and puncture a finger of a person subjected to blood collection with the puncture needle; and
a control part configured to control an operation of the puncture mechanism, wherein
the puncture mechanism is a mechanism that lifts the puncture part from below the finger to puncture the finger with the puncture needle, and
the control part controls the operation of the puncture mechanism to drive rise and fall of the puncture part, and stops the rise of the puncture part by the puncture mechanism by detecting contact of the puncture part with the finger.

2. The blood collection device according to claim 1, wherein
an amount of movement by which the puncture part is lifted by the puncture mechanism is controlled to a predetermined amount of movement set in advance for a plurality of the persons subjected to blood collection.

3. The blood collection device according to claim 2, wherein
the amount of movement by which the puncture part is lifted by the puncture mechanism is controlled to match a distance between the finger placed on a finger placement place and the puncture part at an initial position.

4. The blood collection device according to claim 1, wherein
the puncture mechanism includes a lifting member that is driven to move up and down, a base member that is supported by the lifting member, a movable support member that lifts the puncture part from below, and an elastic member that elastically supports the movable support member to be movable up and down with respect to the base member, and
puncture of the puncture needle to the finger is performed in a state where the movable support member is biased in an upward direction by the elastic member.

5. The blood collection device according to claim 4, wherein
the contact of the puncture part with the finger is detected by detecting lowering of the movable support member against the bias of the elastic member, which is an operation caused by the contact of the puncture part with the finger.

6. The blood collection device according to claim 5, wherein
the lowering of the movable support member is detected by a detection target part that moves up and down together with the movable support member with respect to the base member and a detection sensor that detects that the detection target part moves to a predetermined position.

7. The blood collection device according to claim 1, further comprising:
a holder configured to hold a blood collection tube;
a movement mechanism configured to move the holder to press the blood collection tube against the finger of the person subjected to blood collection; and
a control part configured to control an operation of the movement mechanism, wherein
the movement mechanism is a mechanism that lifts the holder from below the finger to press the blood collection tube against the finger, and
the control part controls the operation of the movement mechanism to drive rise and fall of the holder, and an amount of upward movement of the holder by the movement mechanism is set based on an amount of movement of the puncture part measured when the puncture part is lifted.

8. The blood collection device according to claim 1, further comprising:
a module configured to hold a hemostatic material for hemostasis at a puncture site of the person subjected to blood collection;
a movement mechanism configured to move the module to press the hemostatic material against the finger of the person subjected to blood collection; and
a control part configured to control an operation of the movement mechanism, wherein
the movement mechanism is a mechanism that lifts the module from below the finger to press the hemostatic material against the finger, and
the control part controls the operation of the movement mechanism to drive rise and fall of the module, and an amount of upward movement of the module by the movement mechanism is set based on an amount of movement of the puncture part measured when the puncture part is lifted.

9. The blood collection device according to claim 1, further comprising:
a module configured to hold a protective material for protecting a puncture site of the person subjected to blood collection;
a movement mechanism configured to move the module to press the protective material against the finger of the person subjected to blood collection; and
a control part configured to control an operation of the movement mechanism, wherein
the movement mechanism is a mechanism that lifts the module from below the finger to press the protective material against the finger, and
the control part controls the operation of the movement mechanism to drive rise and fall of the module, and an amount of upward movement of the module by the movement mechanism is set based on an amount of movement of the puncture part measured when the puncture part is lifted.
